# EUROPÄISCHE PATENTSCHRIFT

(11) **EP 2 606 142 B1**
(45) Veröffentlichungstag und Bekanntmachung des Hinweises auf die Patenterteilung: **28.02.2018**
(21) Anmeldenummer: 11743828.3
(22) Anmeldetag: 12.08.2011
(51) Int. Cl.: C12P 21/00, C07K 1/14, C12N 9/64, C12N 9/99

(54) **VERFAHREN ZUR INAKTIVIERUNG VON PROTEASEN IN EINER AUS EINER ZELLKULTUR GEWONNEN FLÜSSIGKEIT DURCH PH ÄNDERUNG**
PROCESS FOR THE INACTIVATION OF PROTEASES IN A FLUID OBTAINED FROM A CELL CULTURE BY PH SHIFT
PROCÉDÉ D'INACTIVATION DE PROTÉASES DANS UN FLUIDE OBTENU À PARTIR D'UNE CULTURE DE CELLULES PAR MODIFICATION DU PH

(30) Priorität: 20.08.2010 EP 10173540
(43) Veröffentlichungstag der Anmeldung: 26.06.2013
(73) Patentinhaber: Boehringer Ingelheim International GmbH, 55216 Ingelheim am Rhein (DE)
(72) Erfinder: JACOBI, Alexander, 55216 Ingelheim am Rhein (DE); AMBROSIUS, Dorothee, 55216 Ingelheim am Rhein (DE); DOBBERTHIEN, Philine, 55216 Ingelheim am Rhein (DE); ECKERMANN, Christian, 55216 Ingelheim am Rhein (DE); NOTHELFER, Franz, 55216 Ingelheim am Rhein (DE)
(74) Vertreter: Simon, Elke Anna Maria
(86) Internationale Anmeldenummer: PCT/EP2011/063915
(87) Internationale Veröffentlichungsnummer: WO 2012/022688

(56) Entgegenhaltungen:
- WO-A2-2004/035608
- WO-A2-2004/043373
- WO-A2-2005/017174

## Beschreibung

### TECHNISCHES GEBIET

Die Erfindung liegt auf dem Gebiet der Herstellung biopharmazeutischer Produkte. Sie betrifft insbesondere die Verbesserung des Prozesses zur Herstellung biopharmazeutischer Produkte durch die Inaktivierung proteolytisch aktiver Enzyme im zellfreien Zellkulturüberstand.

### STAND DER TECHNIK

Biomoleküle wie Proteine, Polynukleotide, Polysaccharide und dergleichen gewinnen als Medikamente, als Diagnostika, als Zusatzstoffe in Nahrungsmitteln, Waschmitteln und dergleichen, als Forschungsreagenzien und für viele weitere Anwendungen zunehmend an kommerzieller Bedeutung. Der Bedarf an solchen Biomolekülen lässt sich - z.B. im Falle von Proteinen - in aller Regel nicht mehr durch Isolierung der Moleküle aus natürlichen Quellen befriedigen sondern erfordert den Einsatz biotechnologischer Produktionsmethoden.

Die biotechnologische Herstellung von Proteinen beginnt typischerweise mit der Klonierung eines DNS-Fragmentes in einen geeigneten Expressionsvektor. Nach Transfektion des Expressionsvektors in geeignete prokaryontische oder eukaryontische Expressionszellen und anschließender Selektion transfizierter Zellen werden letztere in Fermentern kultiviert und das gewünschte Protein zur Expression gebracht. Anschließend erfolgt die Ernte der Zellen bzw. des Kulturüberstandes und die Aufarbeitung und Reinigung des darin enthaltenen Proteins.

Bekannt ist das Vorkommen von Proteasen in der geernteten Flüssigkeit, z.B. in zellfreiem Kulturüberstand. Sowohl Biopharmazeutika wie monoklonale Antikörper oder rekombinante Proteine als auch Chromatographiematerialien wie immobilisiertes Protein A können durch Proteasen sehr rasch degradiert oder in ihrer Struktur geschädigt werden. Einbußen in der Produktqualität (Homogenität, Funktionalität) und bei Chromatographiematerial die Verminderung der Bindekapazität und die damit verbundene Verunreinigung der Produktfraktionen sind die Folge. Insbesondere bei der serumfreien Kultivierung und guter Produktivität der Zellen liegen die produzierten Biopharmazeutika in hohen relativen Konzentrationen vor und sind somit besonders anfällig für proteolytische Schädigung der Molekülstruktur, was sowohl zu verringerter Ausbeute, als auch zu reduzierter Produktqualität führt.

Proteinschädigungen durch Proteasen können bereits bei neutralen pH-Werten auftreten, umfangreiche Proteindegradation kann jedoch insbesondere dann beobachtet werden, wenn für das Reinigungsverfahren der zellfreie Kulturüberstand auf saure pH-Werte eingestellt werden muss, zum Beispiel, um Bindebedingungen für den Fang-Schritt ("Capture"), z.B. Kationen-Austauschchromatographie, einzustellen (Konditionierung).

Bekannt ist die irreversible Inaktivierung einiger Proteasen, z.B. von Verdauungsenzymen wie Pepsin, durch Änderungen des pH-Werts (Z. Bohak, Purification and Characterization of Chicken Pepsinogen and Chicken Pepsin, Journal of Biological Chemistry 244 (17) (1969) 4633-4648; B. Turk, V. Turk, Lysosomes as Suicide Bags in Cell Death: Myth or Reality?, Journal of Biological Chemistry 284 (33) (2009) 21783-21787). Auch die Zugabe von Proteasehemmstoffen ist vorgeschlagen worden (A.J. Barrett, A. A. Kembhavi, M. A. Brown, H. Kirschke, C. G. Knight, M. Tamai and K. Hanada, L-trans-Epoxysuccinyl-leucylamido(4-guanidino)butane (E-64) and its analogues as inhibitors of cysteine proteinases including cathepsins B, H and L, Biochem. J. 201 (1982) 189-198). Diese sind jedoch sehr teuer, giftig und schwer aus dem Produkt wieder zu entfernen. Sie stellen daher keine Option für die ökonomische Herstellung von sicheren Arzneimitteln dar.

Die Patentschrift WO 2004/043373 A2 offenbart ein Verfahren zur Inaktivierung von Proteasen bzw. zur Reduktion der Proteindegradation in zellfreien Flüssigkeiten, die aus Säugerzellkulturen erhalten werden, mit dem Schritt: (a) Einstellen eines pH-Wertes von 4,8 bis 8, bevorzugt von 4,8 bis 5,5, besonders bevorzugt von 4,8 bis 5,2. Das Ziel des Verfahrens ist es, Proteasen zu inaktivieren, um die proteolytische Degradation eines Fermentationsproduktes zu verringern.

### KURZE ZUSAMMENFASSUNG DER ERFINDUNG

Die Erfindung betrifft ein Verfahren zur Inaktivierung von Proteasen durch mehrfachen Wechsel des pH-Wertes im Zellkulturüberstand zu Beginn des Reinigungsprozesses von Biopharmazeutika. Vorteile der Erfindung sind eine Verbesserung von Produktqualität und Produktausbeute, sowie eine längere Haltbarkeit von Chromatographiematerialien.

Überraschender Weise wurde gefunden, dass in den geernteten, zellfreien Fermentations-Überständen von Säugerzelllinien (z.B CHO, "chinese hamster ovary" cells) Proteasen vorhanden sind, die durch einen Wechsel zu sauren pH-Werten aktiviert und durch anschließende Änderung des pH-Werts ins neutrale Milieu auch in ihrer Aktivität am pH-Optimum irreversibel inaktiviert werden können. Proteasen, die bei neutralen pH-Werten aktiv sind, können durch einen Wechsel zu sauren pH-Werten ebenfalls in ihrer Aktivität bei neutralen Bedingungen irreversibel inaktiviert werden.

Die vorliegende Erfindung betrifft ein Verfahren zur Inaktivierung von Proteasen in Flüssigkeiten, die aus Säugerzellkulturen erhalten werden, mit den Schritten:
(a) Einstellen eines pH-Wertes von 3 bis 5 in der Flüssigkeit, und anschließend
(b) Einstellen eines pH-Wertes von 7 bis 9 in der Flüssigkeit,
wobei der Schritt (a) bei einer Temperatur von 20 bis 30°C durchgeführt und der pH-Wert in Schritt (a) für einen Zeitraum von 5 Minuten bis 30 Minuten gehalten wird.

In einem anderen Aspekt betrifft die vorliegende Erfindung ein Verfahren zur Reduktion der Proteindegradation in Flüssigkeiten, die aus Säugerzellkulturen erhalten werden, mit den Schritten:
(a) Einstellen eines pH-Wertes von 3 bis 5 in der Flüssigkeit, und anschließend
(b) Einstellen eines pH-Wertes von 7 bis 9 in der Flüssigkeit,
wobei der Schritt (a) bei einer Temperatur von 20 bis 30°C durchgeführt und der pH-Wert in Schritt (a) für einen Zeitraum von 5 Minuten bis 30 Minuten gehalten wird.

### KURZE BESCHREIBUNG DER ZEICHNUNGEN

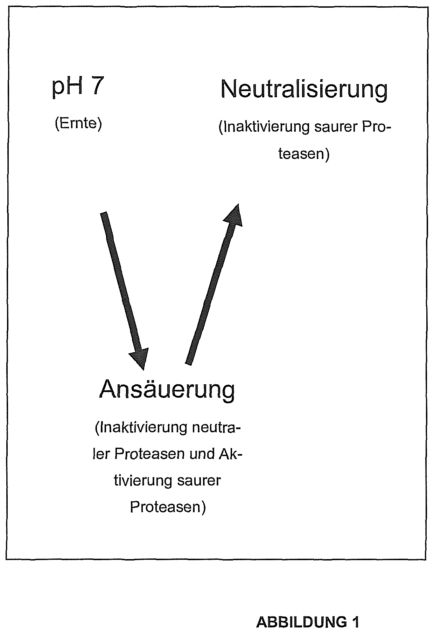

**Abbildung 1****:** Aktivierung und Inaktivierung von Proteasen aus CCF durch zweimalige Veränderung des pH-Wertes.

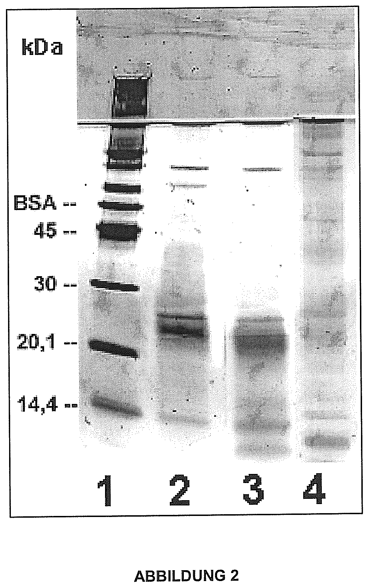

**Abbildung 2****:** Degradation des Modellsubstrates Interferon (IFN) bei saurem pH-Wert. 0,1 mg/ml Interferon wurde mit 10 % (v/v) Zellkulturüberstand (CCF) bei pH 4 für 0 bzw. 14 Stunden mit und ohne pH-Wert-Wechsel (Spur 2 bis 4) bei 37°C inkubiert und mittels SDS-PAGE aufgetrennt. Der pH-Wert-Wechsel wurde bei 20°C mit je 5 minütigen Halteschritten bei pH 4 und bei pH 7. IFN wird durch den pH-Wert-Wechsel deutlich weniger degradiert (Spur 3) als ohne pH-Inaktivierung. Nach 14 Stunden ist IFN vollständig abgebaut (Spur 4).

### Spurbelegung:

1 - Marker
2 - IFN (0,1 mg/mL) vor Inkubation
3 - CCF + IFN pH 4 mit pH-Wert-Wechsel, 14 h Inkubation
4 - CCF + IFN pH 4 ohne pH-Wert-Wechsel 14 h Inkubation

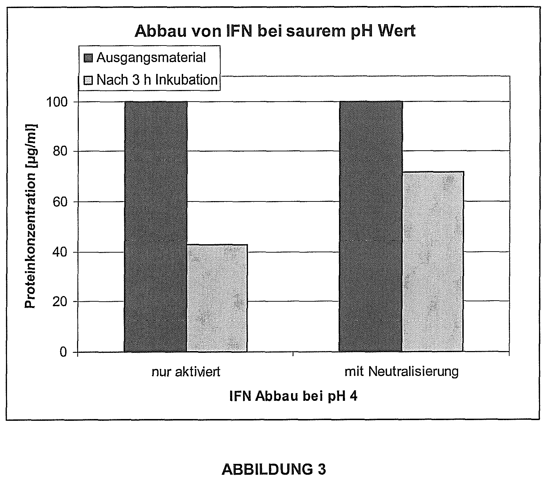

**Abbildung 3****:** Abbau des Proteins IFN durch dreistündige Inkubation mit CCF, 10 % (v/v) bei pH 4.0, Analytik mit RP-HPLC. Nach Inaktivierung der Proteasen durch Neutralisierung und anschließender Inkubation mit IFN bei pH 4.0 sind nach drei Stunden noch 72 % des IFN über RP-HPLC detektierbar, während zum gleichen Zeitpunkt ohne Inaktivierung nur noch 43 % des IFN intakt sind. Die proteolytische Aktivität gegenüber einem nativen Protein konnte somit um die Hälfte reduziert werden.

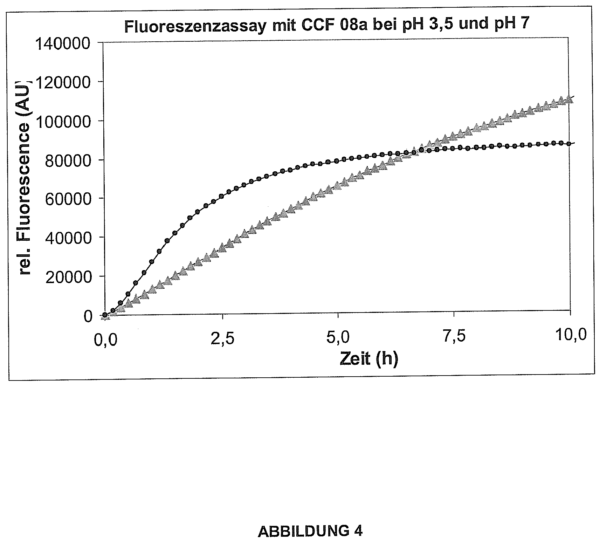

**Abbildung 4****:** Fluoreszenzassay bei saurem und neutralem pH-Wert. Proteasen im CCF sind bei pH 3,5 (●) und bei pH 7 (▲) aktiv. Als Maß der proteolytischen Aktivität durch im CCF vorhandene Proteasen bei pH 3,5 und pH 7 wurde die Freisetzung eines Fluorophors durch Spaltung eines Peptidsubstrates gemessen.

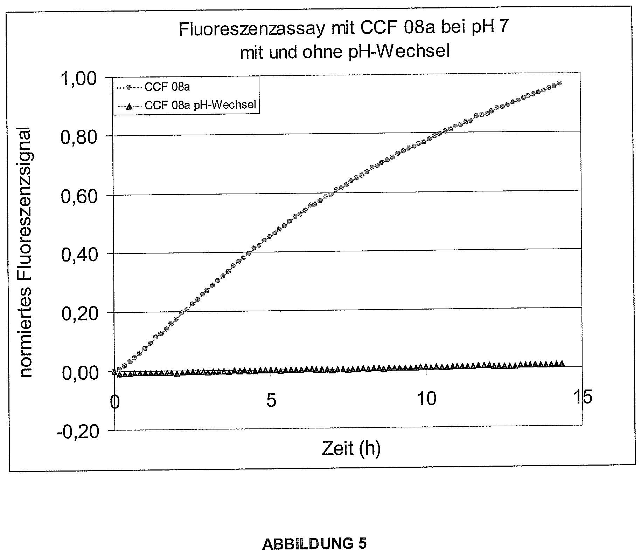

**Abbildung 5****:** Proteolytische Aktivität neutraler Proteasen ohne und mit pH-Wert-Wechsel. Neutrale Proteasen können durch Ansäuerung auf pH < 5 und anschließende Neutralisierung nahezu vollständig und irreversibel inaktiviert werden. Die Messung erfolgte jeweils bei pH 7, als Maß der proteolytischen Aktivität wurde die Freisetzung eines Fluorophors durch Spaltung eines Peptidsubstrates gemessen.

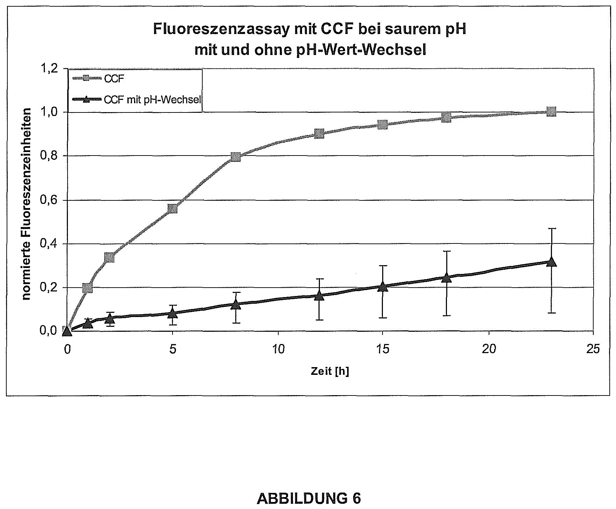

**Abbildung 6****:** Proteolytische Aktivität saurer Proteasen ohne und mit pH-Wert-Wechsel. Die Aktivierung/Inaktivierung der Proteasen im CCF erfolgte durch pH-Wert-Änderung analog Abbildung 1. Aktivierte Proteasen sind bei pH < 5 aktiv und spalten das Substrat. Aktivierte Proteasen, die durch kurze Inkubation bei pH ≥ 7 inaktiviert wurden, zeigen eine auf 35 % verminderte Restaktivität bei pH 3,7. Die Messung erfolgte jeweils bei pH 3,7, als Maß der proteolytischen Aktivität wurde die Freisetzung eines Fluorophors durch Spaltung eines Peptidsubstrates gemessen.

### DETAILLIERTE BESCHREIBUNG DER ERFINDUNG

Die Erfindung betrifft ein Verfahren zur Inaktivierung von Proteasen durch mehrfachen Wechsel des pH-Wertes im Zellkulturüberstand zu Beginn des Reinigungsprozesses. Vorteile der Erfindung sind eine Verbesserung von Produktqualität und Produktausbeute, sowie eine längere Haltbarkeit von Chromatographiematerialien.

Überraschender Weise wurde gefunden, dass in den geernteten, zellfreien Fermentations-Überständen von Säugerzelllinien (z.B CHO, "chinese hamster ovary" cells) Proteasen vorhanden sind, die durch einen Wechsel zu sauren pH-Werten aktiviert und durch anschließende Änderung des pH-Werts ins neutrale Milieu auch in ihrer Aktivität am pH-Optimum irreversibel inaktiviert werden können. Proteasen, die bei neutralen pH-Werten aktiv sind, können durch einen Wechsel zu sauren pH-Werten ebenfalls in ihrer Aktivität bei neutralen Bedingungen irreversibel inaktiviert werden.

Nach Ansäuerung des Zellkulturüberstandes kommt es offenbar zur Aktivierung der enthaltenen Proteasen, was auf das Vorhandensein ursprünglich lysosomaler proteolytischer Enzyme (Cathepsine) hindeutet. Diese Proteasen sind in den Abbau endozytierter Proteine involviert, werden ubiquitär in allen Geweben als nicht-aktive Proformen exprimiert und sind intrazellulär in Endosomen lokalisiert. Die Reifung dieser Kompartimente zu Lysosomen geht mit einer drastischen pH-Wert Erniedrigung einher, die sowohl zu autokatalytischer als auch *in trans* Aktivierung der lysosomalen Proteasen führt. Die Sekretion von Cathepsinen in den extrazellulären Raum wird hauptsächlich im Kontext der Metastasierung von Tumorgewebe diskutiert, für einzelne Cathepsine wurde die Sekretion auch in Zellkultur beschrieben.

Die proteolytische Aktivität bei neutralen pH-Werten ist zum einen auf sezernierte und zum anderen auf ursprünglich membranständige Proteasen zurückzuführen, die vermutlich während des Produktionsprozesses von der Zellmembran getrennt werden und in Lösung weiterhin aktiv sind.

Typischer Weise werden in biopharmazeutischen Prozessen Zellen durch Zentrifugation oder Filtration vom produkthaltigen Zellkulturüberstand separiert. Der zellfreie Überstand wird anschließend sterilfiltriert (max. 0,2 µM Porengröße) und zur Umpufferung vor dem Capture-Schritt diafiltriert. Die Inaktivierung durch doppelte pH-Wert-Änderung kann frühestens direkt nach der Separation des Zellkulturüberstandes von den Zellen erfolgen und bei jeder beliebigen späteren Prozessstufe eingesetzt werden.

Bei der Einstellung der pH-Werte sollte das Produktmolekül und die technischen Anlagen nicht geschädigt werden, pH-Werte < 3 (chemische Modifikation des Produktproteins und verstärkte Korrosion von Stahlbehältern) sind daher zu vermeiden, ebenso pH > 9 (Deamidierung von Asparagin und Glutamin). Auch die Haltezeiten bei den jeweiligen pH-Werten richten sich nach der Stabilität des Produktproteins. Die Zeitspanne für die Aktivierung von Proteasen durch Ansäuerung sollte möglichst kurz sein. Die Haltezeiten sollten zwischen 5 und 30 Minuten, vorzugsweise 5 bis 15 liegen. Bei längeren Haltezeiten zur Aktivierung bei sauren pH-Werten kann es zum verstärkten proteolytischen Abbau des Zielproteins kommen. Die Zeitspanne des anschließenden Halteschritts zur Inaktivierung der sauren Proteasen bei neutralem pH-Wert ist unkritisch und der pH kann auch über Prozessschritte hinweg gehalten bzw. wieder variiert werden, da hier bereits alle neutral-aktiven Proteasen inaktiviert wurden und keine proteolytische Aktivität mehr verzeichnet werden kann. Vorteilhafte Haltezeiten für den Neutralisierungsschritt sind beispielsweise 5 bis 60 Minuten.

Die Einstellung der jeweiligen Ziel-pH-Werte kann in Lösung mittels einmaliger Zugabe oder Titration von Säuren wie Essigsäure oder Salzsäure bzw. Laugen/Basen wie Natronlauge oder Tris unter Rühren erfolgen. Im Säureschritt werden vorteilhaft pH-Werte zwischen 3 und 5 eingestellt, beispielsweise ein pH-Wert von 3,5 bis 4,5, vorzugsweise von 4. Für die anschließende Inaktivierung saurer Proteasen durch Neutralisierung haben sich pH-Werte von 7-9 als effizient erwiesen, vorzugsweise pH 7,4-8,5.

Die Erfindung kann in einem Temperaturbereich von 4°C- 37°C ausgeführt werden, bevorzugt 15°C bis 37°C, bevorzugt 20-37°C. Ein bevorzugter Bereich zur Ausführung der Erfindung ist 20°C bis 30°C.

Das Verfahren der Inaktivierung saurer und neutraler Proteasen durch doppelte pH-Wert-Änderung wurde an zellfreien Kulturüberständen von Säugerzellen (CHO und NS0) erfolgreich durchgeführt. Die Ergebnisse lassen sich auch auf Kulturüberstände anderer Produktionsorganismen übertragen und ist im Rahmen der Anforderungen des Produktproteins, insbesondere dessen pH-Stabilität, in der Herstellung verschiedener biopharmazeutischer Produkte anwendbar.

Die vorliegende Erfindung bedient sich rein physiko- und biochemischer Methoden. Durch zweimalige Änderung des pH-Wertes über mehrere pH-Einheiten (Abbildung 1) kann bis zu 75 % der sauren Proteaseaktivität und bis zu 90% der neutralen Proteaseaktivität irreversibel eliminiert werden. Der Nachweis der Proteaseaktivität kann über zwei Nachweismethoden a) mittels Fluoreszenzfreisetzung nach Peptidspaltung und b) durch Degradation nativer Proteinsubstrate erfolgen.

Produkt- und materialschädigende Proteasen können bei der Herstellung biopharmazeutischer Medikamente durch eine einfache und schnell durchzuführende physikochemische Methode irreversibel inaktiviert werden. Der Zellkulturüberstand ist dadurch variabel einsetzbar und verschiedensten Reinigungstechniken zugänglich.

Die Zugabe von Säuren und Laugen in Tanks ist sehr einfach und schnell durchzuführen und auch in den großtechnischen Maßstab übertragbar. Nach der Inaktivierung der Proteasen kann der Zellkulturüberstand den Reinigungsverfahren sehr variabel angepasst werden. Standzeiten oder pH-Werte sind im Gegensatz zu den üblichen Produktionsprozessen unkritisch.

### Beispiele

### Arbeitsbeispiel 1: pH-Wert-Wechsel nach Ernte, vor Capture Schritt

CHO-Zellen werden im Fed-Batch, 80 L Endvolumen über 11 Tage kultiviert. Der Zellkulturüberstand (CCF) wird auf 20°C temperiert und mittels Durchflusstellerzentrifuge von den Zellen getrennt und über eine Filterkaskade sterilfiltriert. Anschließend wird der pH-Wert des CCF durch Zugabe von Essigsäure auf pH 4 gesenkt, nach Erreichen des Ziel-pH-Wertes wird dieser für 5-10 min gehalten, bevor das CCF durch Zugabe von Natronlauge auf pH 7,5 neutralisiert wird. Vor der weiteren Prozessierung (Capture Schritt) wird das CCF über eine 50 kD MWCO-Membran ultra-/diafiltriert, um geeignete Bindebedingungen für den Capture Schritt einzustellen.

### Arbeitsbeispiel 2: pH-Wert-Wechsel direkt nach der Separation von CCF und Zellen, vor Sterilfiltration

CHO-Zellen werden im Fed-Batch, 80 L Endvolumen über 11 Tage kultiviert. Der Zellkulturüberstand (CCF) wird mittels Durchflusstellerzentrifuge von den Zellen getrennt und auf 20°C temperiert. Anschließend wird der pH-Wert des CCF durch Zugabe von Essigsäure unter ständigem Rühren auf pH 4 gesenkt, nach Erreichen des Ziel-pH-Wertes wird dieser für 5-10 min gehalten, bevor das CCF durch Zugabe von Natronlauge auf pH 7,5 neutralisiert wird. Das behandelte CCF wird anschließend über eine Filterkaskade sterilfiltriert. Vor der weiteren Prozessierung (Capture Schritt) wird das CCF über eine 50 kD MWCO-Membran ultra-/diafiltriert, um geeignete Bindebedingungen für den Capture Schritt einzustellen.

### Arbeitsbeispiel 3: pH-Wert-Wechsel nach rProtA-Capture-Schritt, vor Virusinaktivierung

CHO-Zellen werden im Fed-Batch, 80 L Endvolumen über 11 Tage kultiviert. Der Zellkulturüberstand (CCF) wird mittels Durchflusstellerzentrifuge von den Zellen getrennt, über eine Filterkaskade sterilfiltriert und mittels einer 50 kD MWCO-Membran ultra-/diafiltriert, um geeignete Bindebedingungen für den Capture Schritt, eine rProteinA-Affinitätschromatographie, auf PBS pH 7,5 einzustellen. Eine MabSelect Chromatographiesäule wird mit 32 mg mAb pro mL Säulenmatrix beladen und der Antikörper in einer Stufe mit Acetatpuffer pH 3,5 eluiert. Der pH-Wert der produkthaltigen Fraktion wird durch Zugabe von 1 M Tris unter Rühren auf pH 7,5 eingestellt und der pH für 10 min bei Raumtemperatur gehalten, bevor geeignete Bedingungen für eine saure Virusinaktivierung eingestellt werden.

### Material und Methoden

### Zellkulturüberstand

Auf sekretorische Produktion von therapeutischen Proteinen optimierte Maus- und CHO-Produktionszelllinien werden mehrere Tage in serumfreien Medium kultiviert. Der Zellkulturüberstand (CCF, cell free cell culture fluid) wird durch Filtration oder Zentrifugation von Zellen und unlöslichen Bestandteilen getrennt und nach Einstellung des jeweiligen pH-Wertes mit 10-20 % (v/v) für die Aktivitätsassays verwendet.

### Einstellung des pH-Wertes

Die Ansäuerung der Zellkulturüberstände erfolgt durch Zugabe von Essigsäure. Die Proben werden unverzüglich gemischt und für 5-10 Minuten beim eingestellten pH-Wert inkubiert. Präzipitierende Bestandteile werden durch Zentrifugation pelletiert und verworfen. Die Erhöhung des pH-Wertes erfolgt durch Zugabe von Natronlauge oder 1 M Tris-Base.

### Inhibitionsexperiemente zur Ermittlung der Proteaseklassen

Für die Inhibition einzelner Proteaseklassen wird CCF mit verschiedenen käuflichen Inhibitoren inkubiert und die noch vorhandene Aktivität anschließend in den Aktivitätsassays untersucht. Die Inhibitorkonzentration wird gemäß der Herstellerempfehlung verwendet.

### Aktivitätsassays

### Fluoreszenzassay, Analyse durch Freisetzung eines Fluorophors

Als Substrate für die kinetische und quantitative Bestimmung der proteolytischen Aktivität dienen verschiedene Peptid-Fluorophor-Konjugate, deren Spaltung zur Freisetzung von Fluoreszenzfarbstoffen wie Aminomethylcoumarin (AMC) oder der Aufhebung des Quencheffektes durch Dinitrophenyl (Dnp) auf N-Methylaminobenzoyl-Diaminopropionsäure (Nma) führt. Der Anstieg des Fluoreszenzsignals kann photometrisch bei λ_{Ex}= 380 nm; λ_{Em} = 460 nm (AMC), λ_{Ex} = 340 nm; λ_{Em}=460 nm im Multilabel-Counter Victor^{2,3} (Perkin Elmer, Massachusetts, USA) verfolgt werden (Abb. 3).

Alle Assays für kinetische und quantitative Analysen werden in der Substratsättigung bei 0,2 mM Peptid-AMC, 10-20 µM Peptid-MCA oder 5-10 µM DnP-Peptid-Nma in Gegenwart von 10 - 20 % (v/v) CCF bei pH 7 (100 mM Tris/HCl, 200 mM NaCl) und pH 3,5 (100 mM NaAcetat, 200 mM NaCl) durchgeführt.

### Degradationsassay, Analyse durch PAGE und RP-HPLC

Als Substrat für die qualitative Analyse proteolytischer Aktivität dient ein natives Protein der Interferonfamilie (IFN). IFN hat eine Größe von 22,5 kD und liegt in Lösung monomer vor. Für den Degradationsassay werden 0,1 mg/ml IFN mit 10 % (v/v) CCF bei pH 4 bis zu 24 Stunden bei 37°C inkubiert und mittels SDS-Page und Silberfärbung nach Heukeshoven (Heukeshoven, J., Dernick, R., Improved silver staining procedure for fast staining in PhastSystem Development Unit. I. Staining of sodium dodecyl sulfate gels. Electrophoresis, 9, (1988) 28-32.) detektiert oder per RP-HPLC der Abbau des Proteins als Maß für proteolytische Aktivität bestimmt.

Die RP-HPLC-Analyse erfolgt an einer HPLC-Anlage der Firma Waters (Waters 2695 alliance) mit UV-Detektor (Waters 2487 Dual Absorbance Detector) mittels einer Vydac 214 TP-C₄-Säule durch Gradientenelution von 0,2 % (v/v) TFA in Wasser (Lösung A) zu 0,15 % (v/v) TFA in Acetonitril (Lösung B) (Tabelle 1).

**Tabelle 1: Elutionsgradient für eine RP-HPLC-C4-Säule. Der Gradient verläuft von wässrigem zu organischem Lösungsmittel**

| **Zeit [min]** | **Lösung A [%]** | **Lösung B [%]** |
|---|---|---|
| 5,0 | 60 | 40 |
| 15,0 | 30 | 70 |
| 15,1 | 10 | 90 |
| 19,0 | 10 | 90 |
| 19,1 | 60 | 40 |
| 21,0 | 60 | 40 |

### Ergebnisse

Die Substratspaltung durch Proteasen aus CCF hat zwei Optima, diese liegen bei sauren pH-Werten pH < 5 und im Neutralen um pH 7. Die Aktivitäten der jeweiligen Proteasen können durch Proteinabbau und durch Fluoreszenzfreisetzung nach Spaltung eines fluorogenen Peptidsubstrates verfolgt werden (Abb. 2 und 4). Abbildung 2 zeigt den Abbau von IFN bei pH 4 in Gegenwart von 10 % (v/v) CCF. Bereits nach wenigen Stunden wird IFN bei sauren Bedingungen abgebaut (Lane 4), In Abbildung 4 ist der zeitliche Verlauf der Spaltung des Dnp-Peptid-Nma-Substrates durch 20 % (v/v) CCF nach Inkubation bei pH 3.5 und pH 7 dargestellt. Das steigende Fluoreszenzsignal ist ein Maß für die Spaltung des Peptidsubstrates. Sowohl im Sauren als auch bei neutralen pH-Werten wird Aktivität beobachtet.

Diese Aktivität kann durch Proteaseklassen-spezifische Inhibitoren unterdrückt werden, wodurch nachgewiesen werden kann, dass mehrere Proteaseklassen im CCF vorhanden sind und dass diese in zwei Gruppen geteilt werden können: die sauer-aktiven Proteasen, die nur bei niedrigen pH-Werten, und die neutral-aktiven Proteasen, die nur bei neutralen pH-Werten aktiv sind. Die sauer-aktiven besitzen dabei keine Aktivität bei neutralen pH-Werten und die neutral-aktiven keine bei sauren pH-Werten. Während die neutral-aktiven Proteasen bereits zum Zeitpunkt der Zellseparation aktiv sind, erfolgt die Aktivierung der sauer-aktiven Proteasen in CCF erst durch Ansäuerung der Reaktionsbedingungen.

### Zweistufige pH-Wertänderung zur Inaktivierung neutraler Proteasen

Die Aktivität neutraler Proteasen aus unbehandeltem CCF kann bei pH 7 im Fluoreszenzassay ermittelt werden (Abb. 5, Kreise). Unterzieht man das CCF einer zweistufigen pH-Wertänderung von pH 7 auf pH < 5 und anschließender Neutralisierung lässt sich bei pH 7 im Fluoreszenzassay nahezu keine Aktivität mehr detektieren (Abb. 5, Dreiecke).

Die kurzzeitige Ansäuerung des CCFs und anschließende Neutralisierung führt zum vollständigen und irreversiblen Verlust der proteolytischen Aktivität von neutral-aktiven Proteasen.

### Zweistufige pH-Wertänderung zur Inaktivierung saurer Proteasen

Die proteolytische Aktivität der durch Ansäuerung des CCF aktivierten Proteasen kann bei pH 3,5 im Fluoreszenzassay und im Proteinabbauassay detektiert werden (Abb. 6 Quadrate). Diese Aktivität bleibt jedoch nicht erhalten, wenn das CCF nach der Ansäuerung neutralisiert wird. Werden nach der Neutralisierung die für saure Proteasen optimalen Reaktionsbedingungen von pH 3,5 wieder hergestellt, ist die im Fluoreszenzassay messbare Aktivität der sauren Proteasen um bis zu 65 % gegenüber dem unbehandelten CCF reduziert (Abb. 6, Dreiecke).

Auch die Degradation von Proteinen ist durch die doppelte pH-Wertänderung verringert. Der Abbau des Modellproteins IFN ist nach dem Neutralisationsschritt signifikant reduziert (Abb. 3, graue Balken).

Der Abbau des nativen Proteinsubstrates IFN konnte durch den mehrfachen Wechsel des pH-Wertes um 50 % reduziert werden (Abb. 3, Quantifizierung durch RP-HPLC).

## Patentansprüche

1. Verfahren zur Inaktivierung von Proteasen in Flüssigkeiten, die aus Säugerzellkulturen erhalten werden, mit den Schritten:
(a) Einstellen eines pH-Wertes von 3 bis 5 in der Flüssigkeit, und anschließend
(b) Einstellen eines pH-Wertes von 7 bis 9 in der Flüssigkeit, wobei der Schritt (a) bei einer Temperatur von 20 bis 30°C durchgeführt und der pH-Wert in Schritt (a) für einen Zeitraum von 5 Minuten bis 30 Minuten gehalten wird.

2. Verfahren zur Reduktion der Proteindegradation in Flüssigkeiten, die aus Säugerzellkulturen erhalten werden, mit den Schritten:
(a) Einstellen eines pH-Wertes von 3 bis 5 in der Flüssigkeit, und anschließend
(b) Einstellen eines pH-Wertes von 7 bis 9 in der Flüssigkeit,
wobei der Schritt (a) bei einer Temperatur von 20 bis 30°C durchgeführt und der pH-Wert in Schritt (a) für einen Zeitraum von 5 Minuten bis 30 Minuten gehalten wird.

3. Verfahren nach Anspruch 1 oder 2, **dadurch gekennzeichnet, dass** der pH-Wert in Schritt (a) in einem Bereich von 3,5 bis 4,5 liegt.

4. Verfahren nach einem der Ansprüche 1 bis 3, **dadurch gekennzeichnet, dass** der pH-Wert in Schritt (b) in einem Bereich von 7,4 bis 8,5 liegt.

5. Verfahren nach einem der Ansprüche 1 bis 4, **dadurch gekennzeichnet, dass** der pH-Wert in Schritt (b) für einen Zeitraum von 5 Minuten bis 60 Minuten gehalten wird.

6. Verfahren nach einem der Ansprüche 1 bis 5, **dadurch gekennzeichnet, dass** der Schritt (b) bei einer Temperatur von 20 bis 30°C durchgeführt wird.

7. Verfahren nach einem der Ansprüche 1 bis 6, **dadurch gekennzeichnet, dass** es sich bei der Flüssigkeit um zellfreie Flüssigkeit einer Säugerzellkultur handelt.

## Claims

1. Process for inactivating proteases in liquids that are obtained from mammalian cell cultures, comprising the steps of:
(a) adjusting the pH of the liquid to 3 to 5; and then
(b) adjusting the pH of the liquid to 7 to 9,
wherein step (a) is carried out at a temperature of 20 to 30°C, and the pH in step (a) is maintained for a period of 5 minutes to 30 minutes.

2. Process for reducing the protein degradation in liquids that are obtained from mammalian cell cultures, comprising the steps of:
(a) adjusting the pH of the liquid to 3 to 5; and then
(b) adjusting the pH of the liquid to 7 to 9,
wherein step (a) is carried out at a temperature of 20 to 30°C, and the pH in step (a) is maintained for a period of 5 minutes to 30 minutes.

3. Process according to Claim 1 or 2, **characterised in that** the pH in step (a) is in the range 3.5 to 4.5.

4. Process according to one of Claims 1 to 3, **characterised in that** the pH in step (b) is in the range 7.4 to 8.5.

5. Process according to one of Claims 1 to 4, **characterised in that** the pH in step (b) is maintained for a period of 5 minutes to 60 minutes.

6. Process according to one of Claims 1 to 5, **characterised in that** step (b) is carried out at a temperature of 20 to 30°C.

7. Process according to one of Claims 1 to 6, **characterised in that** the liquid is a cell-free liquid from a mammalian cell culture.

## Revendications

1. Procédé d'inactivation de protéases dans des fluides qui sont obtenus à partir de cultures de cellules de mammifères, avec les étapes suivantes :
(a) ajustement d'un pH de 3 à 5 dans le fluide et ensuite
(b) ajustement d'un pH de 7 à 9 dans le fluide,
dans lequel l'étape (a) est réalisée à une température de 20 à 30 °C et le pH à l'étape (a) est maintenu pendant une durée de 5 minutes à 30 minutes.

2. Procédé de réduction de la dégradation de protéines dans des fluides qui sont obtenus à partir de cultures de cellules de mammifères, avec les étapes suivantes :
(a) ajustement d'un pH de 3 à 5 dans le fluide et ensuite
(b) ajustement d'un pH de 7 à 9 dans le fluide,
dans lequel l'étape (a) est réalisée à une température de 20 à 30 °C et le pH à l'étape (a) est maintenu pendant une durée de 5 minutes à 30 minutes.

3. Procédé selon la revendication 1 ou 2, **caractérisé en ce que** le pH à l'étape (a) se situe dans une plage de 3,5 à 4,5.

4. Procédé selon l'une des revendications 1 à 3, **caractérisé en ce que** le pH à l'étape (b) se situe dans une plage de 7,4 à 8,5.

5. Procédé selon l'une des revendications 1 à 4, **caractérisé en ce que** le pH à l'étape (b) est maintenu pendant une durée de 5 minutes à 60 minutes.

6. Procédé selon l'une des revendications 1 à 5, **caractérisé en ce que** l'étape (b) est réalisée à une température de 20 à 30 °C.

7. Procédé selon l'une des revendications 1 à 6, **caractérisé en ce que** le fluide est un fluide acellulaire d'une culture de cellules de mammifères.
